# EUROPEAN PATENT SPECIFICATION

(11) **EP 2 207 419 B1**
(45) Date of publication and mention of the grant of the patent: **16.10.2013**
(21) Application number: 08844363.5
(22) Date of filing: 30.10.2008
(51) Int. Cl.: A01N 25/16, A01N 25/30, A01N 59/12, A61K 47/06, A61P 31/02, A01P 1/00

(54) **FOAM STABILIZER FOR A STERILIZATION COMPOSITION**
SCHAUMSTOFFSTABILISATOR FÜR EINE STERILISIERUNGSZUSAMMENSETZUNG
STABILISATEUR DE MOUSSE POUR UNE COMPOSITION STERILISANTE

(30) Priority: 31.10.2007 JP 2007283787
(43) Date of publication of application: 21.07.2010
(73) Proprietor: Mundipharma International Limited, Hamilton HMJX (BM)
(72) Inventor: CHIKAKO, Udagawa, Osaka 586-0084 (JP); YOSHIE, Kono, Tokyo 104-8002 (JP); TAKAKO, Okamoto, Yokohama-shi Kanagawa 222-8567 (JP)
(74) Representative: Maiwald, Walter
(86) International application number: PCT/EP2008/009180
(87) International publication number: WO 2009/056322

(56) References cited:
- WO-A-91/12721
- WO-A-03/061389
- WO-A-03/061721
- GB-A- 2 204 054
- US-A- 4 130 640
- US-A- 4 597 975

## Description

### TECHNICAL FIELD

This invention relates to a composition for sterilization capable of forming persistent and stable foam.

### BACKGROUND ART

Various drugs are used as antiseptics for hands, fingers and skin, and povidone-iodine is well known as a disinfectant/antiseptic. The povidone-iodine is used in various sites such as hospitals and homes since the povidone-iodine is known to hardly allow an occurrence of resistant microbe, and to have high disinfection capability on various pathogenic microbes.
As drug products of povidone-iodine, formulations such as an ointment, a paste, or a powder have been developed and used (see Patent Reference 1 or 2, for example), and a liquid drug is mainly used as a formulation for hands and fingers (see Patent Reference 3, for example). Such a liquid drug is a solution having a blackish color derived from iodine and known to have a problem of dripping which has been often pointed out. Particularly, in the liquid drug of cleansing type, dripping and scattering easily occur when dispensing on a hand upon using, and therefore, a part of the drug solution dispensed on a hand is usually wasted. Further, there are many cases where the drug solution is scattered on clothes, floor, and the like, and a considerable time is required for washing and cleaning.
[Patent Reference 1] Japanese Patent Examined Publication No. 1-32210
[Patent Reference 2] Japanese Patent No. 3583166
[Patent Reference 3] Japanese Patent Examined Publication No. 7-2646

WO 03/061721 and WO 03/061389 describe antimicrobial film-forming compositions.

US 4,130,640 describes a germicidal cleaning composition in semi-solid form.

US 4,597,975 describes iodine-containing surface active compositions useful for cleansing and degermining skin.

WO 91/12721 pertains to a bacteriocidal and viricidal disinfectant composition suitable for application to the skin.

### DISCLOSURE OF THE INVENTION

### PROBLEMS TO BE SOLVED BY THE INVENTION

An object of this invention is to provide a composition for sterilization that is prevented from dripping and scattering caused in use and enables effective use of all drug solution dispensed on the hand, with effective disinfection of hands and fingers.

### MEANS FOR SOLVING THE PROBLEMS

The inventors have conducted extensive research in order to solve the above problems and as a result, by blending a specific amount of an organosulfate type surfactant with a specific amount of povidone-iodine and a specific amount of a nonionic surfactant, it is possible to make a formulation for a stable and persistent foamed drug product and to prevent dripping that is caused in use and, as a result, the inventors have found a composition for sterilization that enables effective use of all the drug solution dispensed on the hand, prevention of smirching of clothes, floor, and the like, and at the same time, effective disinfection of hands and fingers, thereby accomplishing this invention.
That is, this invention is as described below.
(1) A composition for sterilization comprising povidone-iodine, a nonionic surfactant and an organosulfate type surfactant, wherein the blending amount of povidone-iodine is 4 to 10 (WN)%, the blending ratio between the organosulfate type surfactant and the nonionic surfactant is from 50:1 to 2:1, and the total of the contents of the two surfactants is 0.4 to 14 (WN)% with respect to the total amount of the composition.
(2) A composition for sterilization according to (1), wherein the organosulfate type surfactant described in (1) is nonylphenyl polyoxyethylene ether ammonium sulfate or lauryl polyoxyethylene ether ammonium sulfate.
(3) A composition for sterilization according to (1), wherein the nonionic surfactant described in (1) is diethanolamide laurate.
(4) A composition for sterilization according to any one of (1) to (3), wherein a blending amount of the organosulfate type surfactant described in (1) is 0.4 to 10 (W/V)%.
(5) A composition for sterilization according to (1), wherein the composition for sterilization has a foaming power of 190 to 270 mm, a foam stability of 140 to 201 mm, and a reduction in foaming power of 30% or less.
(6) The composition for sterilization according to (1), wherein the dripping time is 5 minutes or more.

### EFFECT OF THE INVENTION

A composition for sterilization in this invention enables reliable cleansing and disinfection by preventing dripping and forming persistently stable foam. Further, since the composition for sterilization of this invention does not drip, the complete amount of the composition for sterilization dispensed on the hand is effectively used, and it is possible to improve utilization of an effective component as a drug and economic efficiency.

### BEST MODE FOR CARRYING OUT THE INVENTION

The composition for sterilization in this invention includes povidone-iodine having a disinfection action and serving as a main component, and an organosulfate type surfactant and a nonionic surfactant. If necessary, a pH adjuster, a solvent, and other pharmaceutical additives may be blended.

Since the composition for sterilization in this invention is discharged from a pump type container, it is not particularly necessary to limit viscosity insofar as clogging of a filter inside a nozzle of a pump is prevented. Since excessively high viscosity causes clogging, the viscosity of the composition may preferably be 10 mPa·s or less.

The pH level of the composition for sterilization in this invention may preferably be 3 to 6 in view of stability over time. In this case, it is preferable to adjust the pH of the composition for sterilization with a pH adjuster. As the pH adjuster, sodium hydroxide, hydrochloric acid, or the like may be used.

The concentration of povidone-iodine in this invention is 4 to 10 (W/V)%, more preferably 4 to 7.5 (W/V)% with respect to the total amount of the composition, in view of the viscosity.

The organosulfate type surfactant in this invention may particularly preferably be an organosulfate type surfactant of an ammonium salt, and, as specific examples thereof, nonylphenyl polyoxyethylene ether ammonium sulfate or lauryl polyoxyethylene ether ammonium sulfate may be used. The blending amount of the organosulfate surfactant may preferably be 0.4 to 10 (W/V)% with respect to the total amount of the composition.

As another aspect of this invention, in the case of blending the nonionic surfactant, it is possible to use diethanolamide laurate. In order to form a persistent foam, the amount of the nonionic surfactant blended may preferably be smaller than that of the organosulfate type surfactant blended in this invention. More specifically, the blending ratio of the organosulfate type surfactant and the nonionic surfactant is 50:1 to 2:1, wherein the concentration of the organosulfate type surfactant is 0.4 to 10 (WN)%, and the concentration of the nonionic surfactant is 0.02 to 1 (WN)% with respect to the total amount of the composition for sterilization in this invention. More preferably, the concentration of the organosulfate type surfactant is 1 to 4 (WN)%, and the concentration of the nonionic surfactant is 0.08 to 1 (WN)%. It is possible to form the persistent foam when the concentrations are within these ranges.

The total of the amounts of the organosulfate type surfactant and the nonionic surfactant blended is 0.4 to 14 (W/V)%, preferably 0.4 to 10.2 (W/V)%, with respect to the total amount of the composition for sterilization in this invention.

The composition for sterilization in this invention has a foaming power of 190 to 270 mm at 25°C and a foam stability of 140 to 201 mm at 25°C. A composition for sterilization having a reduction in foaming power of 30% or less when the foaming power and the foam stability are as specified above is preferred. Here, "reduction in foaming power" means a value that is obtained by dividing the foam stability (mm) by the foaming power (mm) and expressed as a percentage (%). The composition for sterilization in this invention having such a value forms persistent and stable foam. Testing methods for the foaming power and the foam stability are described in JIS (Japanese Industrial Standards) K3362:1998 (synthetic detergent testing method), and the tests were conducted in accordance with this method. Specifically, by using a foaming power measurement device described in JIS K3362, 50 mL of a sample is charged in an inner cylinder in advance, and 20 mL of the same sample is dropped on a central part of a liquid surface from a height of 900 mm under a temperature condition of 25°C. After dripping the whole sample, the height (mm) of the foam was measured, and the measured value is the foaming power. Furthermore, the height of the foam after 5 minutes had passed was measured, and the measured value is the foam stability.

The composition for sterilization in this invention does not cause dripping for 3 minutes or more, and more preferably 5 minutes or more. Here, "Time in which dripping does not occur" means the time elapsed until dripping of liquid droplets from the sieve starts as a result of breakage of the foam when the composition for sterilization which has been filled in the pump type container is discharged by pressing the pump once with a hand on a sieve having a screen number of 16 (1 µm) which is defined in the Japanese Pharmacopeia. Such time is referred to as the dripping time (min) of the discharged foam. The foam having the dripping time of 3 minutes or more forms sufficiently persistent foam, and the foam having the dripping time of 5 minutes or more is furthermore finer, more persistent, and more preferred in terms of foam properties.

In the composition for sterilization in this invention, it is possible to use a stabilizer such as potassium iodide, a viscosity adjuster such as lauryl pyrrolidone, hydroxyethylcellulose, and concentrated glycerin, flavoring, if necessary.

As a solvent used in this invention, any solvent may be used as long as povidone-iodine is completely dissolved in the solvent, and specifically, purified water, or distilled water may be used as the solvent. A temperature for dissolution is not particularly limited, and it is ordinarily preferable to perform the dissolution within a temperature range of from 10°C to 30°C.

It is possible to produce the composition for sterilization in this invention by a conventional method. For example, povidone-iodine is added to purified water in an amount of from 70% to 90% of the total amount of the composition for sterilization in this invention, followed by sufficient stirring for perfect dissolution, and then the organosulfate type surfactant is added, followed by sufficient stirring for perfect dissolution. A pH level of the solution is adjusted by using the pH adjuster. Purified water is added to the solution after the pH adjustment to achieve the predetermined concentration, whereby producing the composition for sterilization.

Any container may be used as the pump type container to be charged with the composition for sterilization in this invention insofar as the container is provided with a pump that makes the composition for sterilization into foam without scattering. Therefore, for example, a pump foamer container capable of discharging foam when a nozzle part thereof is pressed down may be used. Further, a mesh filter or a porous filter is necessarily attached inside the nozzle of the pump. In the case of using a material subject to solidification easily by drying, such as povidone-iodine, it is more preferable to use the porous filter than to use the mesh filter. As specific examples of products, pump foamer containers E3 type, F5L type, WRT4 type, manufactured by Daiwa Can Company, a non-gas type pump foamer container manufactured by Yoshino Kogyosho Co., Ltd., and a pump foam dispenser PF03F type manufactured by Kohno Jushi Kogyo Group may be used.

As used herein, the mesh filter means a filter in the form of a mesh. A material is not particularly limited, and it is possible to use PET (polyethylenetelephthalate), PP (polypropylene). The size of the mesh at the time is not particularly limited, and it is possible to select appropriately in accordance with the purpose. For example, the size may preferably be 100-mesh or more, more preferably 100- to 400-mesh, and further preferably 200- to 350-mesh. The number of filters is not particularly limited and may be selected appropriately in accordance with the purpose. From the viewpoint of improving the foam property, two or more filters may preferably be used for example. Further, a thickness of the filter is not particularly limited and may be selected appropriately in accordance with the purpose. The thickness may preferably be 0.01 to 2 mm.

Further, the porous filter means a filter having fine pores. It is possible to define the fine pores by way of the number of cells (density). The number of cells (density) of a porous body may preferably be 10 to 200 cells/25 mm, and more preferably 25 to 175 cells/25mm. A material is not also particularly limited, and CFS (polyphenylene sulfite), urethane, PP (polypropylene) may preferably be used. It is also possible to use filters different in mesh and different materials in combination. Further, a thickness of the filter is not particularly limited and may be selected appropriately in accordance with the purpose. For example, the thickness may preferably be I to 10 mm. Specifically, the porous body used for such as the pump of WO2006/131980 may be used.

### Examples

Hereinafter, this invention will be described in more detail by way of examples, however, this invention is not limited to the examples and test examples.

### Examples 1 to 6 and Comparative Examples 1 and 2

To purified water of 25°C, povidone-iodine and diethanolamide laurate that had been melted were added based on the blending amount of Table 1, followed by mixing. Furthermore, nonylphenyl polyoxyethylene ether ammonium sulfate was added to the mixture, followed by perfect dissolution. A pH level of each of the solutions was adjusted to 4.2, and then purified water was added to each of the solutions to achieve a total amount of 100 mL, thereby obtaining compositions for sterilization in this invention.
In the same manner, Comparative Examples 1 and 2 were prepared based on the blending amount of Table 1.

### Examples 7 to 12 and Comparative Examples 3 and 4.

To purified water of 25°C, povidone-iodine and diethanolamide laurate that had been melted were added based on the blending amount of Table 2, followed by mixing. Further, lauryl polyoxyethylene ether ammonium sulfate was added to the mixture, followed by perfect dissolution. A pH level of each of the solutions was adjusted to 4.2, and then purified water was added to each of the solutions to achieve a total amount of 100 mL, thereby obtaining compositions for sterilization in this invention.
In the same manner, Comparative Examples 3 and 4 were prepared based on the blending amount of Table 2.

### Test Example 1 Dripping Test

Each of the compositions for sterilization (hereinafter referred to as samples: formulations are shown in Tables 1 to 4) of this invention different in components from one another was charged in a pump type container of 350 mL (E3-08 type manufactured by Daiwa Can Company; discharge volume of 20 cm³). The pump was pressed down once with a hand, and the discharged foam was dropped on a sieve of the Japanese Pharmacopeia sieve number 16 (1 µm) to measure a time elapsed till the foam was deformed to cause droplets to start falling down from the sieve. The time was taken as a dripping time (min) of the discharged foam. The results are shown in Tables 1 and 2. The compositions for sterilization in this invention were capable of maintaining fine foam for 5 minutes or more and preventing from dripping from the sieve in this test, but foams of the compositions within the range outside this invention were rapidly disappeared to cause dripping.

### Foaming Power Test

Foaming power of the samples were measured in accordance with the column "Foaming Power and Foam Stability" described in the synthetic detergent testing method of JIS K3362. Specifically, by using a foaming power measurement device described in JIS K3362, 50 mL of each of the samples was charged in an inner cylinder in advance, and 20 mL of the same sample was dropped on a central part of a liquid surface from a height of 900 mm under a temperature condition of 25°C. After dripping whole of the sample, a height (mm) of the foam was measured, and the measured value was taken as the foaming power. Furthermore, a height of the foam after 5 minutes had passed was measured, and the measured value was taken as the foam stability. This test was conducted for each of the samples in the same manner. The results are shown in Tables 1 and 2.

**TABLE 1**

| | | Example | | | | | | Comparative Example | |
|---|---|---|---|---|---|---|---|---|---|
| | | 1 | 2 | 3 | 4 | 5 | 6 | 1 | 2 |
| Blended components | Povidone-iodine (g) | 7.5 | 7.5 | 7.5 | 7.5 | 7.5 | 7.5 | 7.5 | 7.5 |
| | a: Nonylphenyl polyoxyethylene ether ammonium sulfate (g) | 0.5 | 2 | 1 | 3.5 | 1 | 10 | 0.5 | 2.5 |
| | b: Diethanolamide laurate (g) | 0.25 | 1 | 0.2 | 0.25 | 0.02 | 0.2 | 1 | 5 |
| | Blending ratio (a:b) | 2:1 | 2:1 | 5:1 | 14:1 | 50:1 | 50:1 | 0.5:1 | 0.5:1 |

| | Sodium hydroxide (g) | Adequate amount | Adequate amount | Adequate amount | Adequate amount | Adequate amount | Adequate amount | Adequate amount | Adequate amount |
|---|---|---|---|---|---|---|---|---|---|
| | Purified water (mL) | Added so that total amount reaches to 100 ml | | | | | | | |
| Foaming power (mm) | | 245 | 270 | 245 | 245 | 215 | 205 | 175 | 155 |
| Foam stability (mm) | | 184 | 201 | 180 | 181 | 163 | 149 | 143 | 120 |
| Dripping time (min) | | 5 minutes or more | 5 minutes or more | 5 minutes or more | 5 minutes or more | 5 minutes or more | 5 minutes | 3 seconds | 25 seconds |

**TABLE 2**

| | | Example | | | | | | Comparative Example | |
|---|---|---|---|---|---|---|---|---|---|
| | | 7 | 8 | 9 | 10 | 11 | 12 | 3 | 4 |
| Blended components | Povidone-iodine (g) | 7.5 | 7.5 | 7.5 | 7.5 | 7.5 | 7.5 | 7.5 | 7.5 |
| | a: Lauryl polyoxyethylene ether ammonium sulfate (g) | 0.5 | 2 | 1 | 4 | 1 | 10 | 1.25 | 2.5 |
| | b: Diethanolamide laurate (g) | 0.25 | 1 | 0.1 | 0.4 | 0.02 | 0.2 | 2.5 | 5 |
| | Blending ratio (a:b) | 2:1 | 2:1 | 10:1 | 10:1 | 50:1 | 50:1 | 0.5:1 | 0.5:1 |
| | Sodium hydroxide (g) | Adequate amount | Adequate amount | Adequate amount | Adequate amount | Adequate amount | Adequate amount | Adequate amount | Adequate amount |
| | Purified water (mL) | Added so that total amount reaches to 100 ml | | | | | | | |
| Foaming power (mm) | | 250 | 270 | 260 | 245 | 245 | 240 | 190 | 160 |
| Foam stability (mm) | | 195 | 196 | 200 | 196 | 191 | 164 | 150 | 120 |
| Dripping time (min) | 5 minutes or more | 5 minutes or more | 5 minutes or more | 5 minutes or more | 5 minutes or more | 5 minutes | 4 seconds | 25 seconds | |

### INDUSTRIAL APPLICABILITY

The composition for sterilization in this invention is excellent in forming a persistent foam and usable for effective disinfection and sterilization.

## Claims

1. A composition for sterilization comprising povidone-iodine, a nonionic surfactant and an organosulfate type surfactant, wherein the blending amount of povidone-iodine is 4 to 10 (W/V)%, the blending ratio between the organosulfate type surfactant and the nonionic surfactant is from 50:1 to 2:1, and the total of the contents of the two surfactants is 0.4 to 14 (WN)% with respect to the total amount of the composition.

2. A composition for sterilization according to claim 1, wherein the organosulfate type surfactant is nonylphenyl polyoxyethylene ether ammonium sulfate or lauryl polyoxyethylene ether ammonium sulfate.

3. A composition for sterilization according to claim 1, wherein the nonionic surfactant is diethanolamide laurate.

4. A composition for sterilization according to any preceding claim, wherein the blending amount of the organosulfate type surfactant described in claim 1 is 0.4 to 10 (W/V)%.

5. A composition for sterilization according to claim 1, wherein the composition for sterilization has a foaming power of 190 to 270 mm, a foam stability of 140 to 201 mm, and a reduction in foaming power of 30% or less.

## Patentansprüche

1. Eine Zusammensetzung zur Sterilisierung enthaltend Povidon-Jod, ein nichtionisches Tensid und ein Tensid des Organosulfat-Typs, worin die enthaltene Menge Povidon-Jod 4 bis 10 Gew.-%, das Mischungsverhältnis zwischen dem Tensid vom Organosulfat-Typ und dem nichtionischen Tensid von 50:1 bis 2:1, und der Gesamtgehalt der beiden Tenside 0,4 bis 14 Gew.-% beträgt in Bezug auf die Gesamtmenge der Zusammensetzung.

2. Eine Zusammensetzung zur Sterilisierung gemäß Anspruch 1, worin das Tensid vom Organosulfat-Typ Nonylphenyl, Polyoxyethylenetherammoniumsulfat oder Lauryl-polyoxyethylenetherammoniumsulfat ist.

3. Eine Zusammensetzung zur Sterilisierung gemäß Anspruch 1, worin das nichtionische Tensid Diethanolaminlaurat ist.

4. Eine Zusammensetzung zur Sterilisierung gemäß irgendeinem der vorhergehenden Ansprüche, worin die enthaltende Menge des Tensids vom Organosulfat-Typ gemäß Anspruch 1 0,4 bis 10 Gew.-% beträgt.

5. Eine Zusammensetzung zur Sterilisierung gemäß Anspruch 1, wobei die Zusammensetzung zur Sterilisierung eine Schäumkraft von 190 bis 270 ml, eine Schäumkraft von 140 bis 201 ml, und eine Reduktion der Schäumkraft von 30 % oder weniger hat.

## Revendications

1. Composition de stérilisation comprenant de la povidone iodée, un tensioactif non ionique et un tensioactif de type organosulfate, dans laquelle la quantité de mélange de la povidone iodée va de 4 à 10 % (P/V), le ratio de mélange entre le tensioactif de type organosulfate et le tensioactif non ionique va de 50/1 à 2/1, et le total des teneurs relatives aux deux tensioactifs va de 0,4 à 14 % (P/V) par rapport à la quantité totale de la composition.

2. Composition de stérilisation selon la revendication 1, dans laquelle le tensioactif de type organosulfate est le nonylphényle polyoxoéthylène éther de sulfate d'ammonium ou le lauryl polyoxoéthylène ether de sulfate d'ammonium.

3. Composition de stérilisation selon la revendication 1, dans laquelle le tensioactif non ionique est le diéthanolamide laurique.

4. Composition de stérilisation selon l'une quelconque des revendications précédentes, dans laquelle la quantité de mélange du tensioactif de type organosulfate décrit dans la revendication 1 va de 0,4 à 10 % (P/V).

5. Composition de stérilisation selon la revendication 1, dans laquelle la composition de stérilisation a un pouvoir moussant de 190 à 270 mm, une stabilité de la mousse de 140 à 201 mm et une réduction du pouvoir moussant de 30 % ou moins.
